# EUROPEAN PATENT SPECIFICATION

(11) **EP 0 473 671 B1**
(45) Date of publication and mention of the grant of the patent: **24.08.1994**
(21) Application number: 90908385.9
(22) Date of filing: 02.05.1990
(51) Int. Cl.: A61K 31/155

(54) **USE OF SIGMA-RECEPTORS LIGANDS FOR THE MANUFACTURE OF AN ANXIOLYTIC AGENT**
VERWENDUNG VON SIGMA-REZEPTOR-LIGANDEN ZUR HERSTELLUNG EINES ANXIOLYTIKUMS
UTILISATION DE LIGANDS DES RECEPTEURS SIGMA POUR FABRIQUER UN ANXIOLYTIQUE

(30) Priority: 02.05.1989 US 346494
(43) Date of publication of application: 11.03.1992
(73) Proprietor: STATE OF OREGON BY AND THROUGH OREGON STATE BOARD OF HIGHER EDUCATION ON BEHALF OREGON HEALTH SC. UNIV. AND OREGON STATE UNIV., Portland, Oregon 97201 (US)
(72) Inventor: KEANA, John, F., W., Eugene, OR 97405 (US); WEBER, Eckard, Portland, OR 97229 (US)
(74) Representative: Sheard, Andrew Gregory
(86) International application number: US9002398
(87) International publication number: WO9014067

(56) References cited:
- US-A- 4 709 094
- TINS, Volume 10, Number 11 page 461-463 (1987), File, S.E., "The Search for Novel Anxiolytics" See page 461, column 1.
- TINS, Volume 10, Number 1 page 37-40 (1988), Sonders, M.S. et al. "Phenylcyclidine and psycotomimetic sigma opiates: recent insights into their biochemical and physiological sites of action".
- Merck Manual 14th edition

## Description

### Field of the Invention

The invention relates to the use of pharmaceutical compositions comprising an anxiolytic amount of an N,N'-disubstituted guanidine for the manufacture of a medicament for the treatment or prevention of anxiety in animals.

### Background of the Invention

Fear, or apprehension, is characterized by the anticipation of a known danger or event. In contrast, neurotic anxiety is characterized by an apprehension with no known cause, or a maladaptive response to a trivial danger. In recent years, generalized anxiety disorder (GAD) has been characterized by psychiatrists as being chronic (continually present for at least 1 month) and exemplified by three of four psychomotor symptoms: motor tension, autonomic hyperactivity, apprehensive expectation, and vigilance and scanning. Before this characterization was adopted, clinical trials of anxiolytic agents in the United States occurred in patients which were described variously as suffering from anxiety neurosis, anxiety with associated depression, and other such terms. Anxiety disorders affect 2-3% of the general population (the 67 million prescriptions written in 1977 for just two popular anxiolytics confirm this projected incidence). The popularity of anxiolytics attests to their ability to ameliorate the debilitating symptoms of the disease. Taylor, D.P., FASEB J. 2: 2445-2452 (1988).

Historically, anxiety has been treated by agents including alcohol, opiates, and belladonna, which have a sedative component to their action. In the 20th century novel chemical entities were discovered which are safer for the treatment of anxiety including barbiturates, propanediol carbamates, and benzodiazepines. The pharmacological profiles of these drugs have suggested that their actions are mediated by receptors for γ-aminobutyric acid (GABA). Although the benzodiazepines present a safer alternative than meprobamate and phenobarbital, they also are sedatives. In addition, the benzodiazepines control convulsions and produce muscle relaxation, properties that are unneeded or undesirable in tie treatment of anxiety. Furthermore, these drugs can interact with alcohol, with potentially disastrous consequences. Recently, it has been appreciated that the benzodiazepines produce habituation and possess a pronounced liability, for example, withdrawal symptoms after chronic use. The need for anxioselective drugs that are more selective, have fewer side effects, and present a profile consistent with safety during protracted treatment has resulted in a continuing search for such drugs. This search has led to the synthesis and evaluation of agents that possess no obvious homology with the benzodiazepines. Taylor et al., supra.

Buspirone (Buspar) was the first novel anxiolytic to be approved for clinical use in the United States since the benzodiazepines were introduced almost 30 years ago. The introduction of buspirone into clinical trials for the treatment of anxiety was a direct result of its efficacy in a predictive animal model for the disease-- the taming of the aggressive response of rhesus monkeys to the introduction of foreign objects into their cages according to the protocol described by Tompkins, E.C. et al., Res Commun. Psychol. Psychiatry Behav. 5: 337-352 (1980). See Taylor et al., supra.

The preclinical screening of putative anxiolytics is dependant upon animal tests. Most of the laboratory data on new putative anxiolytics come from animal tests from two main classes. The first group of tests are based on conflict or conditioned fear. The second group of tests are based upon anxiety generated by novel situations. Although these tests differ in the way anxiety is produced, there has been surprising agreement amongst them in the classification of drugs as anxiolytic or anxiogenic. See File, S.E., TINS 10:461-463 (1987).

In two particular tests for anxiolytic activity, it is assumed that the anticipation of punishment causes a reduction in a response associated with the punishment. Conversely, anxiolytic agents that reduce anxiety result in an increased response rate. In the Geller-Seifter test, the rat receives food reward for pressing a lever, but also receives an electric footshock, which has the effect of suppressing the response. This punished schedule alternates with an unpunished schedule wherein electric footshocks are not administered. During this unpunished schedule, lever-pressing is still rewarded. In the Vogel test, a rat is allowed to drink water, but also receives an electric shock through the water spout or the bars of the floor. In both the Vogel and Geller-Seifter tests, a measure of unpunished response is obtained in order to allow assessment of any non-specific stimulant or sedative drug effects or any changes in food or water intake. In both of these tests, benzodiazepines enhance the response rate in the punished periods, without increasing the rate of response in the absence of shock. While these tests are valid tests of anxiety, the only means of assessing them has been pharmacological. Taylor et al., supra.

A less widely used test utilizes punished locomotion, wherein a measure of unpunished crossing is obtained according to the rate at which a mouse crosses from one metal plate to another, wherein footshocks are administered whenever the mouse crosses. Although less widely utilized to test anxiolytic agents, this test has been able to detect drug-induced increases and decreases in anxiety by manipulating the shock level. File, S.E., J. Neurosci Methods 2: 219-238 (1980).

The social interaction test of anxiety (File, supra; Jones, B.J. et al., Br. J. Pharmacol. 93: 985-993 (1988) exploits the uncertainty and anxiety generated by placing rats in an unfamiliar environment and in bright light. The dependent variable is the time that pairs of male rats spend in active social interaction (90% of the behaviors are investigatory in nature). Both the familiarity and the light level of the test arena may be manipulated. Undrugged rats show the highest level of social interaction when the test arena is familiar and is lit by low light. Social interaction declines if the arena is unfamiliar to the rats or is lit by bright light. Anxiolytic agents prevent this decline. The overall level of motor activity may also be measured to allow detection of drug effects specific to social behaviors.

The social interaction test of anxiety is one of the few animal tests of anxiety that has been validated behaviorally. Other behavioral measures indicative of anxiety and stress (e.g. defecation, self-grooming and displacement activities) were correlated with the reductions in social interaction; and other causes of response change (e.g. exploration of the environment, odor changes) were excluded. In order to validate the test physiologically, ACTH and corticosterone levels and changes in hypothalamic noradrenaline also were measured. File, TINS 10: 461-463 (1987).

Another test of anxiety that exploits the anxiety generated by a novel situation is the elevated plus maze. In this test, the anxiety is generated by placing the animals on an elevated open arm. Height, rather than the light level, is responsible for generating behavioral and physiological changes. The apparatus is in the shape of a plus with two open and two enclosed arms. The rat has free access to all arms on the apparatus. Anxiolytic activity may be measured by the percentage increase in the time that the test animal spends on the open arms and the number of entries onto the open arms. This test has also been validated behaviorally and physiologically.

Other agents which have been determined to have anxiolytic activity include the carbazole derivative 9-[3-(3,5-cis-dimethylpiperazino)-propyl]carbazole having the Formula (I): 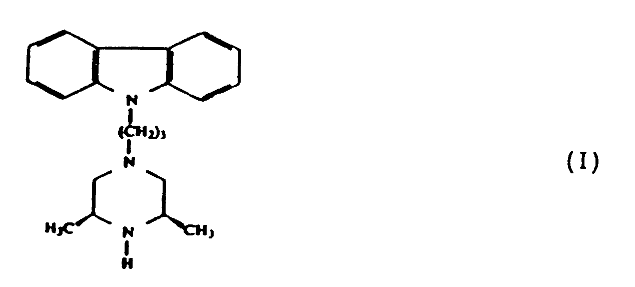
and pharmaceutical compositions thereof. See U.S.-A-4,400,383 (1983).

Serotonin receptor antagonists are also known to be useful for the treatment of anxiety. See Kahn, R.S. et al., J. Affective Disord. 8:197-200 (1987); Westenberg, H.G.M. et al., Psychopharmacol. Bull. 23:146-149 (1987).

Recently, the inventors have described a series of di-arylguanidines which are potent ligands for brain sigma receptors (Weber, et al., PNAS (USA) 83:8784-8788 (1986); Campbell et al., J. Neurosci, in press (1989); U.S.-A-4,709,094). Brain sigma receptors bind many psychotropic drugs (Sonders et al., Trends Neurosci. 11:37-40 (1988)). The physiological function of sigma receptors in the nervous system is subject to intense investigations (Sonders et al., Trends Neurosci. 11:37-40 (1988)) because certain sigma receptor selective compounds have known anti psychotic activity suggesting that sigma receptor active compounds can be used for the treatment of schizophrenia (Largent et al., Eur. J. Pharmacol. 11:345-347 (1988)).

A wide variety of substituted guanidines are disclosed in the patent literature. For example:
US-A-1,411,731 and 1,422,506 discloses diphenylguanidine as a rubber accelerator;
US-A-1,597,233 discloses N-o-tolyl-N'-phenyl-guanidine as a rubber accelerator;
US-A-1,672,431 discloses N,N'-di-o-methoxyphenyl-guanidine as being useful for therapeutic purposes, especially in the form of water-soluble salts;
US-A-1,730,338 discloses N-p-dimethyl-amino-phenyl-N'-phenylguanidine as a rubber accelerator;
US-A-1,795,738 discloses a process for the production of N,N'-dialkyl-di-substituted guanidines, including N-di-ethyl-N'-phenyl-guanidine, N-diethyl-N-isoamylguanidine, N-dimethyl-N'-isoamylguanidine and N-dimethyl-N'-ethylguanidine;
US-A-1,850,682 discloses a process for the preparation of disubstituted guanidine rubber accelerators bearing an additional substituent on the imine nitrogen atom;
US-A-2,145,214 discloses the use of disubstituted guanidines, e.g., diarylguanidines especially dixylylguanidine, as parasiticides;
US-A-2,254,009 discloses sym-di-2-octyl-guanidine and 2,274,476 and 2,289,542 disclose sym-dicyclohexylguanidine as insecticides and moth larvae repellents;
US-A-2,633,474 discloses 1,3-bis(o-ethylphenyl)guanidine and 1,3-bis(p-ethylphenyl)guanidine as rubber accelerators;
US-A-3,117,994 discloses N,N',N''-trisubstituted guanidines and their salts as bacteriostatic compounds;
US-A-3,140,231 discloses N-methyl- and N-ethyl-N'-octylguanidines and their salts as antihypertensive agents;
US-A-3,248,246 describes (Example 5) a 1,3-disubstituted guanidine whose substituents are hydrophobic hydrocarbon groups, one of which is naphthylmethyl and the other is n-butyl;
US-A-3,252,816 discloses various N-substituted and unsubstituted cinnamyl-guanidines and generically the corresponding N'- and N''-alkyl substituted compounds and their salts as antihypertensive agents;
US-A-3,270,054 discloses N-2-adamant-1-yl- and N-2-homoadamant-1-yl-oxy-ethyl-thioethyl- and aminoethyl-guanidine derivatives bearing at most two lower alkyl groups on the N'- and/or N''-nitrogen atom as sympathicolytic and anti-viral agents;
US-A-3,301,755 discloses N-ethylenically unsubstituted-alkyl-guanidines and the corresponding N'- and/or N''-lower alkyl compounds as hypoglycemic and antihypertensive agents;
US-A-3,409,669 discloses N-cyclohexylamino-(3,3-dialkyl-substituted-propyl)-guanidines and the corresponding N'-alkyl- and/or N''-alkyl-substituted compounds as hypotensive agents;
US-A-3,547,951 discloses 1,3-dioxolan-4-yl-alkyl-substituted guanidines which have anti-hypertensive activity and discloses lower alkyl, including n-butyl, as a possible substituent on the other amino group;
US-A-3,639,477 discloses propoxylguanidine compounds as having anorectic properties;
US-A-3,681,459; 3,769,427; 3,803,324; 3,908,013; 3,976,787; and 4,014,934 disclose aromatic substituted guanidine derivatives wherein the phenyl ring can contain hydroxy and/or halogen substituents for use in vasoconstrictive therapy;
US-A-3,804,898 discloses N-benzylcyclobutenyl and N-benzylcyclobutenyl-alkyl-guanidines and the corresponding N-alkyl and/or N''-alkyl-substituted compounds as hypotensive agents;
US-A-3,968,243 discloses N-aralkyl substituted guanidines and the corresponding N'-alkyl-n''alkyl and N',N'-aralkyl compounds as being useful in the treatment of cardiac arrhythmias;
US-A-3,795,533 discloses o-halo-benzylidene-amino-guanidines and their use as anti-depressants for overcoming psychic depression;
US-A-4,007,181 discloses various N,N'-disubstituted guanidines substituted on the imine nitrogen atom by adamantyl as possessing antiarrhythmic and diuretic activities;
US-A-4,051,256 discloses N-phenyl- and N-pyridyl-N'-cycloalkylguanidines as antiviral agents;
US-A-4,052,455 and 4,130,663 disclose styrylamidines, as analgesics agents or for the prevention of blood platelet aggregation;
US-A-4,109,014 discloses N-hydroxysubstituted guanidines and the corresponding N-methyl disubstituted guanidines as vasoconstrictor agents;
US-A-4,169,154 discloses the use of guanidines in the treatment of depression;
US-A-4,393,007 discloses N-substituted and unsubstituted, N-substituted methyl-N'-unsubstituted, monosubstituted and disubstituted-N''-unsubstituted and substituted guanidines as ganglionic blocking agents; and
US-A-4,471,137 discloses N,N,N'N''-tetraalkyl guanidines as being sterically hindered bases useful in chemical synthesis.
US-A-4,709,094 discloses 1,3-disubstituted-guanidines, e.g., 1-3-dibutyl-guanidine and 1,3 di-o-tolyl-guinidine, as sigma brain receptor ligands.

For examples of other substituted guanidines, see, e.g., US-A-1,422,506; 1,642,180; 1,756,315; 3,159,676; 3,228,975; 3,248,426; 3,283,003; 3,320,229; 3,479,437; 3,547,951; 3,639,477; 3,784,643; 3,949,089; 3,975,533; 4,060,640 and 4,161,541.

Geluk, H.W., et al., J. Med. Chem., 12,712 (1969) describe the synthesis of a variety of adamantyl disubstituted guanidines as possible antiviral agents, including N,N'-di-(adamantan-1-yl)-guanidine hydrochloride, N-(adamantan-1-yl)-N'-cyclohexyl-guanidine hydrochloride and N-(adamantan-1-yl)-N'-benzyl-guanidine hydrochloride.

U.S.-A-4,709,094 (1987), discloses N,N'-disubstituted guanidine derivatives which exhibit high binding activity with respect to the sigma receptor having the Formula (II): 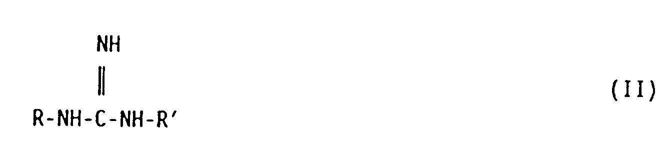
wherein R and R' are an alkyl group of at least 4 carbon atoms, a cycloalkyl group of 3-12 carbon atoms, or carbocyclic or aryl, of at least 6 carbon atoms.

### Summary of the Invention

It has been discovered that certain N,N'-disubstituted guanidines are potent anxiolytics, and at the same time, are substantially non-sedative in an animal model. Therefore, these N,N'-disubstituted guanidines are useful for the treatment or prophylaxis of anxiety in animals, i.e., humans.

The invention is related to the use of an anxiolytic amount of an N,N'-disubstituted guanidine which exhibits a high affinity for the sigma receptor for the manufacture of an anxiolytic agent. These N,N'-disubstituted guanidines exhibit anxiolytic activity in an animal model.

The N,N'-disubsituted guanidines useful for the treatment or prophylaxis of anxiety have the Formula (II): 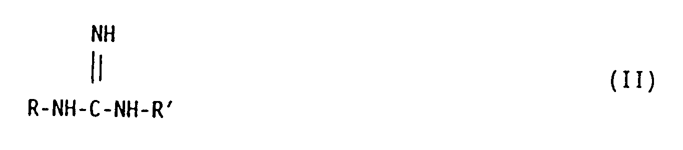
wherein R and R' are an alkyl group of at least 4 carbon atoms, a cycloalkyl group of at least 3 carbon atoms, a carbocyclic aryl group of at least 6 carbon atoms, alkaryl or aralkyl of at least 6 carbon atoms and containing 1-3 separate or fused rings, a heterocyclic ring, and wherein each of R and R' may be substituted in 1-3 positions, or wherein R and R' together with the guanidine group to which they are attached form a cyclic ring containing at least 2 carbon atoms exclusive of the guanidine carbon atom, and wherein said cyclic ring may be substituted with one or more alkyl groups of 1-6 carbon atoms, carbocyclic aryl groups of at least 6 carbon atoms, cycloalkyl groups of 3-12 carbon atoms, or 1-2 fused aromatic rings, and further wherein said N,N'-disubstituted guanidine exhibits a high affinity for the sigma receptor.

### Brief Description of the Drawings

Figure 1 is a graphical representation of the data resulting from the in vivo subcutaneous (s.c.) administration of diazepam on mouse behavior in the black:white test box.

Figure 2 is a graphical representation of the data resulting from the in vivo s.c. administration of N,N'-di(adamant-1-yl)guanidine (DAG) on mouse behavior in the black:white test box.

Figure 3 is a graphical representation of the data resulting from the in vivo s.c. administration of N-(o-tolyl)-N'-(adamant-1-yl)guanidine (AdTG) on mouse behavior in the black:white test box.

Figure 4 is a graphical representation of the data resulting from the in vivo oral administration of AdTG on mouse behavior in the black:white test box.

Figure 5 is a graphical representation of the data resulting from the in vivo s.c. administration of N-(2-iodophenyl)-N'-(adamant-1-yl)guanidine (AdIpG) on mouse behavior in the black:white test box.

Figure 6 is a graphical representation of the data resulting from the in vivo s.c. administration of N-(adamant-1-yl)-N'-[(E)-2-phenylethenyl]phenylguanidine (F-114-B) on mouse behavior in the black:white test box.

Figure 7 is a graphical representation of the data resulting from the in vivo s.c. administration of DTG on mouse behavior in the black:white test box.

Figure 8 is a graphical representation of the data resulting from the oral administration of N,N-di-adamantylguanidine and N-adamantyl-N'-(o-iodophenyl)guanidine, in comparison with controls, on mouse behavior in the black:white test box.

Figure 9 is a graphical representation of the data resulting from the in vivo i.p. administration of diazepam and AdTG (for comparison) in the rat social interaction test.

### Description of the Preferred Embodiments

The invention is related to the discovery that N,N'-disubstituted guanidines having a high affinity for the sigma receptor are anxiolytic, and at the same time, are substantially non-sedative in an animal model. The term "high affinity to sigma receptor" means the compound exhibits an IC₅₀ of less than 100 nM in a sigma receptor binding assay, preferably against ³H-DTG as disclosed in Example 1, below. Alternatively, the compounds may be tested against (+)-[³H]3-PPP as described by Largent, B.L., et al., Mol. Pharmacol. 32:772-784 (1987); Largent B.L., et al., Eur. J. Pharmacol. 155:345-347 (1988); and Wikstrom, H., et al., J. Med. Chem. 30:2169-2174 (1987). The values of IC₅₀ obtained by screening against ³H-DTG and (+)-[³H]3-PPP are well correlated. See Weber, E. et al., Proc. Natl. Acad. Sci. USA 83: 8784-8788 (1986).

These N,N'-disubstituted guanidines exhibit anxiolytic activities at least 100-1000 times greater than that of benzodiazepines. However, unlike benzodiazepines, the N,N'-disubstituted guanidines employed in this invention are non-sedative. Therefore, these N,N'-disubstituted guanidines are particularly useful for the treatment or prevention of anxiety in animals.

Recent work by the inventors has shown that sigma receptor active drugs including the diaryl-guanidines can block acetylcholine release induced by serotonin acting at 5HT₃ receptors in the guinea pig ileum myenteric plexus (Campbell et al., J. Neurosci. in press). The sigma receptor active drugs act in a non-competitive manner to block acetylcholine release stimulated by 5HT₃ receptor activation. Work by others has shown that compounds acting as competitive antagonists at 5HT₃ receptors have anxiolytic activity (Jones et al., Br. J. Pharmacol. 93:985-993 (1988)). Therefore, the inventors reasoned that non-competitive antagonists of 5HT receptor-induced acetylcholine release might also be anxiolytic. This invention shows that certain sigma receptor active N,N'-disubstituted guanidines indeed have potent anxiolytic activity.

The N,N'-disubsituted guanidines useful for the treatment or prophylaxis of anxiety have the Formula (II): 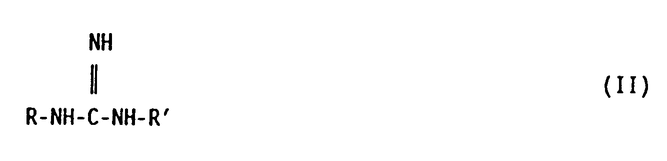
wherein R and R' are an alkyl group of at least 4 carbon atoms, a cycloalkyl group of at least 3 carbon atoms, a carbocyclic aryl group of at least 6 carbon atoms, alkaryl or aralkyl of at least 6 carbon atoms and containing 1-3 separate or fused rings, a heterocyclic ring, and wherein each of R and R' may be substituted in 1-3 positions, or wherein R and R' together with the guanidine group to which they are attached form a cyclic ring containing at least 2 carbon atoms exclusive of the guanidine carbon atom, and wherein said cyclic ring may be substituted with one or more alkyl groups of 1-6 carbon atoms, carbocyclic aryl groups of at least 6 carbon atoms, cycloalkyl groups of 3-12 carbon atoms, or 1-2 fused aromatic rings, and further wherein said N,N'-disubstituted guanidine exhibits a high affinity for the sigma receptor or exhibits anxiolytic activity in an animal model.

Preferred N,N'-disubstituted guanidines which are useful in the practice of this invention are those wherein R and R', which need not be identical, are an alkyl group of at least 4 carbon atoms or carbocyclic aryl groups of at least 6 carbon atoms, e.g., R and R', which can be the same or different, are alkyl of 4 or more carbon atoms, e.g., a 4 to 12 carbon atom, preferably a straight chain alkyl group and more preferably a 4 to 8 carbon atom alkyl group, for example, butyl, isobutyl, tert-butyl, amyl, hexyl, octyl, nonyl and decyl; cycloalkyl of 3 to 12 carbon atoms, e.g., cyclopropyl, cyclopentyl, cyclohexyl, cycloheptyl, 1,4-methylene-cyclohexane, adamantyl, cyclopentylmethyl, cyclohexylmethyl, 1- or 2-cyclohexylethyl and 1-, 2- or 3-cyclohexylpropyl; carbocyclic aryl, alkaryl or aralkyl, e.g., of up to 18 carbon atoms and containing 1-3 separate or fused aromatic rings, e.g., phenyl, benzyl, 1- and 2-phenylethyl, 1-, 2-, or 3-phenylpropyl; o-, m-, or p-tolyl, m,m'-dimethylphenyl, o-, m-, or p-ethylphenyl, m,m'-diethylphenyl, m-methyl-m'-ethylphenyl and o-propylphenyl, naphthyl, 2-naphthyl, and biphenyl, and heterocyclic aromatic rings including pyridyl, pyrazinyl, pyrimidyl, furyl, pyrolyl, thienyl, thiazolyl, oxazolyl, imidazolyl, indolyl, and benzothiazolyl.

Additionally, 1, 2, 3 or more substituents which are chemically and physiologically substantially inert compared to the guanidine group may be present on the R and R' hydrocarbon groups, e.g., alkyl of 1-8 carbon atoms, e.g., methyl, ethyl; halo, e.g., chloro, bromo, iodo, fluoro; nitro; azido; cyano; isocyano; amino; lower-alkylamino; di-lower-alkylamino; trifluoromethyl; alkoxy of 1-8 carbon atoms, e.g., methoxy, ethoxy and propoxy; acyloxy, e.g., alkanoyloxy of 1-8 carbon atoms, e.g., acetoxy and benzoxy; amido, e.g., acetamido, N-ethylacetamido; carbamido, e.g., carbamyl, N-methylcarbamyl, N,N'-dimethylcarbamyl.

A preferred class of compounds of Formula II are those wherein R and R', which need not be necessarily identical, are phenyl or substituted phenyl groups, cyclohexyl, norbornyl, adamant-1-yl and adamant-2-yl groups. Substituted phenyl groups may have one or more of the foregoing substituents, for example, in the o-, m- or p-position or the o-, p- or m, m'-position, when the phenyl group is disubstituted, or R is as herein defined and R' is adamantyl.

Especially preferred anxiolytic guanidine compounds which may be used in the practice of this invention include N-(2-iodophenyl)-N'-(adamant-1-yl)guanidine (AdIpG, IC₅₀ = 6.2 nM); N-(o-tolyl)-N'-(adamant-1-yl)guanidine (AdTG, IC₅₀ = 7.6 nM); N,N'-di(adamant-1-yl)guanidine (DAG, IC₅₀ = 16.5 nM); N-cyclohexyl-N'-(2-methylphenyl)-guanidine (IC₅₀ = 13 nM); N-(adamant-1-yl)-N'-cyclohexylguanidine (IC₅₀ = 12.5 nM); N-adamant-2-yl)-N'-(2-iodophenyl)guanidine (IC₅₀ = 3.5 nM); N-(adamant-2-yl)-N'-(2-methylphenyl)guanidine (IC₅₀ = 7.0 nM); N-(exo-2-norbornyl)-N'-(2-methylphenyl)guanidine (IC₅₀ = 7.0 nM); N-((±)-endo-2-norbornyl)-N'-(2-methylphenyl)guanidine (IC₅₀ = 6.0 nM); N-(exo-2-norbornyl)-N'-(2-iodophenyl)guanidine (IC₅₀ = 4.0 nM); and N-((±)-endo-2-norbornyl)-N'-(2-iodophenyl)guanidine (IC₅₀ = 5.0 nM).

The above-listed compounds are disubstituted guanidines, the class of compounds of which are the subject of U.S.-A-4,709,094, As a class, these compounds are described in this patent as exhibiting a highly selective binding activity to the sigma brain receptor. It has now been determined that certain specific members of this class of disubstituted guanidines, i.e., those demonstrating a high affinity for the sigma receptor, are useful for the treatment or prophylaxis of anxiety in an individual susceptible to anxiety. Individuals susceptible to anxiety are those who have experienced a plurality of prior episodes of GAD.

The anxiolytic activity of any particular N,N'-disubstituted guanidine may be determined by use of any of the recognized animal models for anxiety. A preferred model is described by Jones, B.J. et al., Br. J. Pharmacol. 93:985-993 (1988). This model involves administering the compound in question to mice which have a high basal level of anxiety. The test is based on the finding that such mice find it adversive when taken from a dark home environment in a dark testing room and placed in an area which is painted white and brightly lit. The test box has two compartments, one white and brightly illuminated and one black and non-illuminated. The mouse has access to both compartments via an opening at floor level in the divider between the two compartments. The mice are placed in the center of the brightly illuminated area. After locating the opening to the dark area, the mice are free to pass back and forth between the two compartments. Control mice tend to spend a larger proportion of time in the dark compartment. When given an anxiolytic agent, the mice spend more time exploring the more novel brightly lit compartment and exhibit a delayed latency to move to the dark compartment. Moreover, the mice treated with the anxiolytic agent exhibit more behavior in the white compartment, as measured by exploratory rearings and line crossings. Since the mice can habituate to the test situation, naive mice should always be used in the test. Five parameters may be measured: the latency to entry into the dark compartment, the time spent in each area, the number of transitions between compartments, the number of lines crossed in each compartment, and the number of rears in each compartment. As disclosed more fully below in Example 2, the administration of several N,N-disubstituted guanidines has been found to result in the mice spending more time in the larger, brightly lit area of the test chamber. Unlike diazepam, the N,N-disubstituted guanidines did not cause significant decreases in the numbers of line crossings and rears. Thus, these N,N'-disubstituted guanidines exhibit potent anxiolytic activity, and at the same time, are nonsedating.

In the light/dark exploration model, the anxiolytic activity of a putative agent can be identified by the increase of the numbers of line crossings and rears in the light compartment at the expense of the numbers of line crossings and rears in the dark compartment, in comparison with control mice.

A second preferred animal model is the rat social interaction test described by Jones, B.J. et al., supra, wherein the time that two mice spend in social interaction is quantified. The anxiolytic activity of a putative agent can be identified by the increase in the time that pairs of male rats spend in active social interaction (90% of the behaviors are investigatory in nature). Both the familiarity and the light level of the test arena may be manipulated. Undrugged rats show the highest level of social interaction when the test arena is familiar and is lit by low light. Social interaction declines if the arena is unfamiliar to the rats or is lit by bright light. Anxiolytic agents prevent this decline. The overall level of motor activity may also be measured to allow detection of drug effects specific to social behaviors.

The N,N'-disubstituted guanidines can readily be prepared by conventional chemical reactions, e.g., when R and R' are the same, by reaction of the corresponding amine with cyanogen bromide. Other methods which can be employed include the reaction of an amine with a preformed alkyl or aryl cyanamide. See Safer, S.R., et al., J. Org. Chem. 13:924 (1948). This is the method of choice for producing N,N'-disubstituted guanidines in which the substituents are not identical. For a recent synthesis of unsymmetrical guanidines, see G.J. Durant et al., J. Med. Chem. 28:1414 (1985), and C.A. Maryanoff et al., J. Org. Chem. 51:1882 (1986).

The N,N'-disubstituted guanidines may be administered by any means that achieve their intended purpose. For example, administration may be by parenteral, subcutaneous, intravenous, intramuscular, intra-peritoneal, transdermal, or buccal routes. Alternatively, or concurrently, administration may be by the oral route. The dosage administered will be dependent upon the age, health, and weight of the recipient, kind of concurrent treatment, if any, frequency of treatment, and the nature of the effect desired.

Typically, the anxiolytic compounds may be administered to mammals, e.g. humans, orally at a dose of 0.0025 to 15 mg/kg, or an equivalent amount of the pharmaceutically acceptable salt thereof, per day of the body weight of the mammal being treated for anxiety disorders, e.g., generalized anxiety disorder, phobic disorders, obsessional compulsive disorder, panic disorder, and post traumatic stress disorders. Preferably, 0.01 to 10 mg/kg is orally administered to treat or prevent such disorders. For intramuscular injection, the dose is generally one-half of the oral dose. For example, for treatment or prevention of anxiety, a suitable intramuscular dose would be 0.0025 to 15 mg/kg, and most preferably, from 0.01 to 10 mg/kg.

The unit oral dose may comprise from 0.25 to 400 mg, preferably 0.25 to 100 mg of the anxiolytic compound. The unit dose may be administered one or more times daily as one or more tablets each containing from 0.10 to 300, conveniently 0.25 or 50 mg or the anxiolytic compound or its solvates.

In addition to administering the anxiolytic compound as a raw chemical, the anxiolytic compounds may be administered as part of a pharmaceutical preparation containing suitable pharmaceutically acceptable carriers comprising excipients and auxiliaries which facilitate processing of the anxiolytic compounds into preparations which can be used pharmaceutically. Preferably, the preparations, particularly those preparations which can be administered orally and which can be used for the preferred type of administration, such as tablets, dragees, and capsules, and also preparations which can be administered rectally, such as suppositories, as well as suitable solutions for administration by injection or orally, contain from 0.01 to 99 percent, preferably from 0.25 to 75 percent of active compound(s), together with the excipient.

The pharmaceutical preparations are manufactured in a manner which is itself known, for example, by means of conventional mixing, granulating, dragee-making, dissolving, or lyophilizing processes. Thus, pharmaceutical preparations for oral use can be obtained by combining the active compounds with solid excipients, optionally grinding the resulting mixture and processing the mixture of granules, after adding suitable auxiliaries, if desired or necessary, to obtain tablets or dragee cores.

Suitable excipients are, in particular, fillers such as saccharides, for example lactose or sucrose, mannitol or sorbitol, cellulose preparations and/or calcium phosphates, for example tricalcium phosphate or calcium hydrogen phosphate, as well as binders such as starch paste, using, for example, maize starch, wheat starch, rice starch, potato starch, gelatin, tragacanth, methyl cellulose, hydroxypropylmethylcellulose, sodium carboxymethylcellulose, and/or polyvinyl pyrrolidone. If desired, disintegrating agents may be added such as the above-mentioned starches and also carboxymethyl-starch, cross-linked polyvinyl pyrrolidone, agar, or alginic acid or a salt thereof, such as sodium alginate. Auxiliaries are, above all, flow-regulating agents and lubricants, for example, silica, talc, steric acid or salts thereof, such as magnesium stearate or calcium stearate, and/or polyethylene glycol. Dragee cores are provided with suitable coatings which, if desired, are resistant to gastric juices. For this purpose, concentrated saccharide solutions may be used, which may optionally contain gum arabic, talc, polyvinyl pyrrolidone, polyethylene glycol and/or titanium dioxide, lacquer solutions and suitable organic solvents or solvent mixtures. In order to produce coatings resistant to gastric juices, solutions of suitable cellulose preparations such as acetylcellulose phthalate or hydroxypropymethylcellulose phthalate, are used. Dye stuffs or pigments may be added to the tablets or dragee coatings, for example, for identification or in order to characterize combinations of active compound doses.

Other pharmaceutical preparations which can be used orally include push-fit capsules made of gelatin, as well as soft, sealed capsules made of gelatin and a plasticizer such as glycerol or sorbitol. The push-fit capsules can contain the active compounds in the form of granules which may be mixed with fillers such as lactose, binders such as starches, and/or lubricants such as talc or magnesium stearate and, optionally, stabilizers. In soft capsules, the active compounds are preferably dissolved or suspended in suitable liquids, such as fatty oils, or liquid paraffin. In addition, stabilizers may be added.

Possible pharmaceutical preparations which can be used rectally include, for example, suppositories, which consist of a combination of the active compounds with a suppository base. Suitable suppository bases are, for example, natural or synthetic triglycerides, or paraffin hydrocarbons. In addition, it is also possible to use gelatin rectal capsules which consist of a combination of the active compounds with a base. Possible base materials include, for example, liquid triglycerides, polyethylene glycols, or paraffin hydrocarbons.

Suitable formulations for parenteral administration include aqueous solutions of the active compounds in water-soluble form, for example, water-soluble salts. In addition, suspensions of the active compounds as appropriate oily injection suspensions may be administered. Suitable lipophilic solvents or vehicles include fatty oils, for example, sesame oil, or synthetic fatty acid esters, for example, ethyl oleate or triglycerides. Aqueous injection suspensions may contain substances which increase the viscosity of the suspension include, for example, sodium carboxymethyl cellulose, sorbitol, and/or dextran. Optionally, the suspension may also contain stabilizers.

### EXAMPLES

### Example 1 Sigma receptor binding assays

Sigma receptor binding assays using guinea pig brain membrane homogenates and the radioligands [³H]DTG and (+)[³H]3-PPP were done as previously described (Weber et al., P.N.A.S. (USA) 83:8784-8788 (1986)). Briefly, frozen whole guinea-pig brains (Biotrol, Indianapolis, IN) were homogenized in 10 volumes (w/v) of ice-cold 320 mM sucrose using a Brinkman polytron. The homogenate was centrifuged at 1,000 x g for 20 minutes at 4°C. The supernatant was centrifuged at 20,000 x g for 20 minutes at 4°C. The resulting pellet was resuspended in 10 initial volumes of 50 mM Tris/HCl buffer at pH 7.4 and centrifuged at 20,000 x g for 20 minutes at 4°C. The resulting pellet was resuspended in 5 initial volumes ice-cold 50mM Tris/Hcl (pH .4), and the final volume was adjusted to yield a protein concentration of 3 mg/ml, as determined by dye-binding protein assay (Biorad) using BSA as the standard. Aliquots of 20-ml were stored at -70°C until used, with no detectable loss of binding.

For [³H]DTG binding assays, 20-ml aliquots of the frozen membrane suspension were thawed and diluted 1:3 in 50 mM Tris/HCl (pH 7.4). To 12 x 75 mm polystyrene test tubes were added 0.8 ml of diluted membrane suspension, 0.1 ml of [³H]DTG (46 Ci/mmol; see Weber et al., P.N.A.S (USA) 83 :8784-8788 (1986) or (+)[³H]3-PPP (NEN, 98 Ci/mmol) to yield a final concentration of 1.4 nM, and 0.1 ml of unlabelled drugs or buffer. The protein concentration in the 1-ml final incubation volume was 800 ug/ml, corresponding to 32 mg of brain tissue (original wet weight) and to a tissue concentration within the linear range for specific binding. Non-specific binding was defined as that remaining in the presence of 10 uM haloperidol. Specific binding constituted >90% of total [³H]DTG binding. Incubations were terminated after 90 minutes at room temperature by addition of 4 ml of ice-cold 50mM Tris/HCl (pH 7.4) and rapid filtration of the membrane suspension through Whatman GF/B glass-fiber filters under vacuum, using a 48-well cell harvester (Brandel, Gaithersburg, MD). The filters were washed 2 times with 4 ml of 50 mM Tris/HCl (pH 7.4). Total filtration and washing time was less than 20 seconds. Each filter was dissolved in 10 ml Cytoscint (Westchem, San Diego, CA), and radioactivity was measured by liquid scintillation spectrometry at a counting efficiency of approximately 50%. IC₅₀ values were determined by interpolation from displacement-curve plots on semilogarithmic graph paper.

The IC₅₀ binding values (nM) are as follows: N,N-di(o-tolyl)-guanidine (DTG, 28.0±1); N-(2-iodophenyl)-N'-(adamant-1-yl)guanidine (AdIpG, 6.2 ± 0.7); N-(o-tolyl)-N'-(adamant-1-yl)guanidine (AdTG, 7.6 ± 0.3); N,N'-di(adamant-1-yl)guanidine (DAG, 11.8 ± 3.4); N-(cyclohexyl)-N'-(adamant-1-yl)guanidine (AdChG, 12.5 ± 2.2); N-(o-tolyl)-N'-(cyclohexyl)guanidine (DChG, 86.0 ± 17); N,N'-di-(2,6-dimethylphenyl)-guanidine (DXG, 90 ± 18); N-(o-tolyl)-N'-(4-amino-2-methyl)-phenyl)guanidine (NH₂-DTG, 280 ± 20); N,N'-di(phenyl)guanidine (DPG, 397 ± 21); 2-imino-1,3H-dibenzo[d,f]-[1,3]-diazepine (Bridge-DPG, >100,000); N,N'-di(methyl)guanidine (DMG, >100,000); (+)-3-PPP (76 ± 4); (-)-3-PPP (280 ± 21); (+)-pentazocine (43 ± 2); (-)-pentazocine (135 ± 3); (-)-cyclazocine (2600 ± 210); (-)-SKF10047 (4000 ± 566); haloperidol (5 ± 0.3); BMY 14802 (120 ± 15); rimcazole (1400 ± 100); tiospirone (233 ± 52); perphenazine (42 ± 10); chlorpromazine (1475 ± 265); sulpiride (>100.000); TCP (1100 ± 110); PCP (1050 ± 106); and MK-801 (>10,000).

Nine of these compounds were found to be potent ligands of sigma receptors as determined by their ability to displace [³H]DTG from sigma receptors in guinea-pig brain homogenates, the most potent being AdIpG with an IC₅₀ of 6.2 ± 0.7 nM (n=3).

### Example 2 Effect of Diazepam and N,N'-Disubstituted Guanidines on Light/Dark Exploration of Mice

The inventors wish to thank Brenda Costall, University of Bradford, Bradford, BD7 1DP, England, for screening the sigma receptor ligands according to the procedure disclosed by her and others in Jones et al., Br. J. Pharmacol. 93:985-993 (1988). According to this procedure, male albino BKW mice, 25-30 g, were housed 10 to a cage and allowed free access to food and water. They were kept on reversed light cycle with the lights on between 22 h 00 min and 10 h 00 min.

The apparatus was an open-topped box, 45 cm long, 27 cm wide and 27 cm high, divided into a small (2/5) area and a large (3/5) area by a partition that extended 20 cm above the walls. There was a 7.5 x 7.5 opening in the partition at floor level. The small compartment was painted black and the large compartment white. The floor of each of the compartments was marked into 9 cm squares. The white compartment was illuminated by a 100W tungsten bulb 17 cm above the box and the black compartment by a similarly placed 60W red bulb. The laboratory was illuminated with red light.

All tests were performed between 13 h 00 min and 18 h 00 min. Each mouse was tested by placing it in the center of the white area and allowing it to explore the novel environment for 5 min. Its behavior was recorded on videotape and the behavioral analysis was performed subsequently from the recording. Five parameters were measured: the latency to entry into the dark compartment, the time spent in each area, the number of transitions between compartments, the number of lines crossed in each compartment and the number of rears in each compartment.

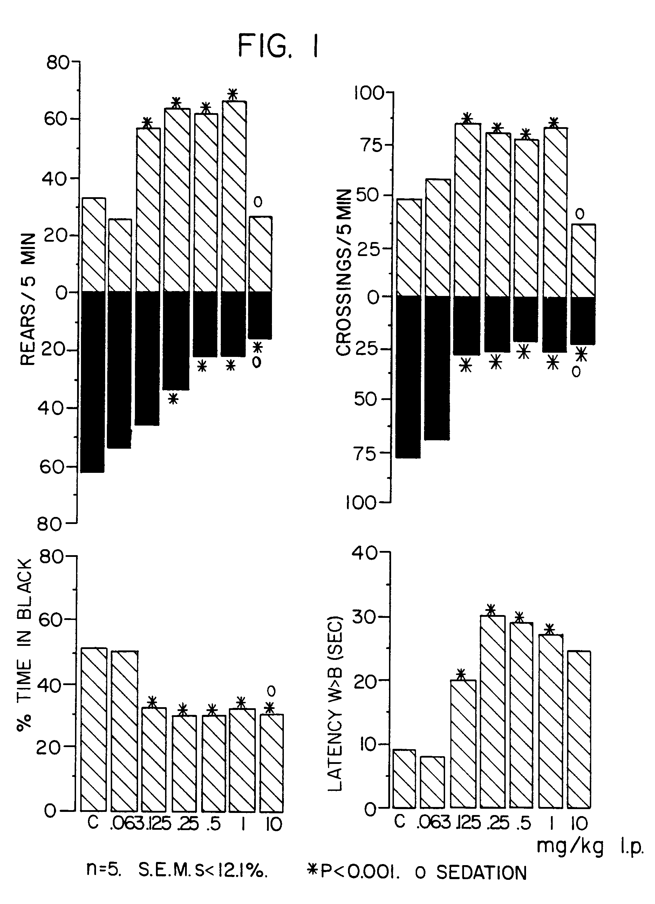

The N,N'-disubstituted guanidines, dissolved in distilled water, were administered subcutaneously 40 min before testing over the dosage range of 0.01 ug to 0.1 mg/kg (5 mice per dosage level). Diazepam (0.063-10 mg/kg) was dissolved in the minimum quantity of polyethylene glycol, diluted to the appropriate volume with distilled water and administered intraperitoneally to five mice for each dosage level. The results are shown in Figures 1-6 (The cross hatched area = light area; solid columns = dark area).

As shown in Figure 1, diazepam dose-dependently increased the proportion of time the mice spent in the larger, lighted area of the test chamber. The numbers of line crossings and rears in the light compartment increased at the expense of those in the dark compartment. At the highest dose of diazepam (10 mg/kg), the numbers of rears and line crossings decreased significantly showing that the drug was markedly sedative. The latency to entering the dark compartment increased with a peak effect at 0.25 mg/kg (latency = 30 sec) while controls showed a latency of 9 sec (S.E.M.s < 12.1%, P<0.001).

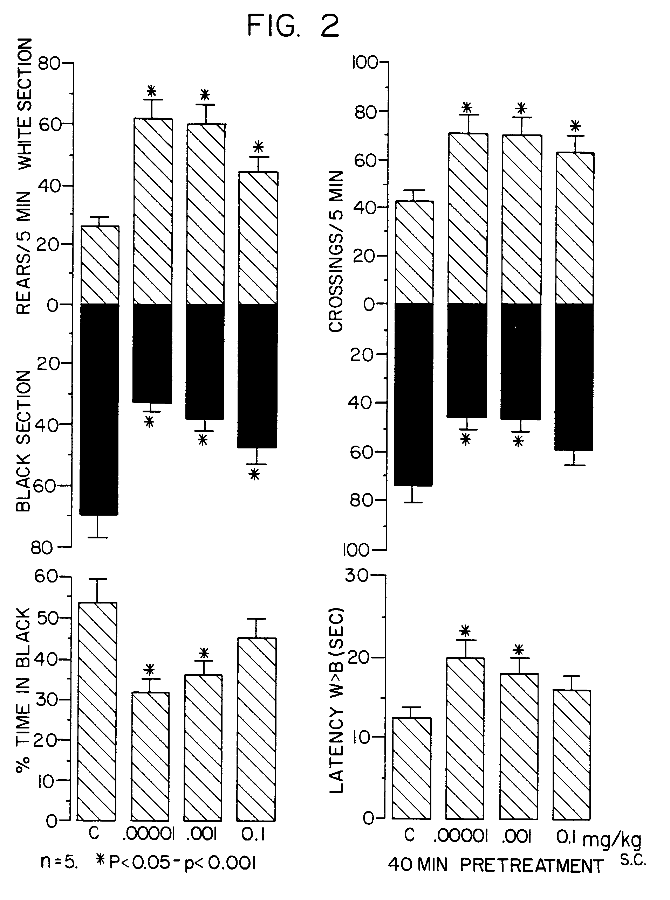

As shown in Figure 2, N,N'-di-adamantylguanidine tended to increase the proportion of time the mice spent in the larger, lighted area of the test chamber, with a peak at 0.01 ug/kg. The numbers of line crossings and rears in the light compartment increased at the expense of those in the dark compartment. At the highest dose of N,N'-di-adamantyl guanidine (0.1 mg/kg), the numbers of rears and line crossings did not decrease significantly showing that the drug was not sedative at this level. The latency to entering the dark compartment increased with a peak effect at 0.01 ug/kg (latency = 20 sec) while controls showed a latency of 12 sec ( P<0.05<0.001).

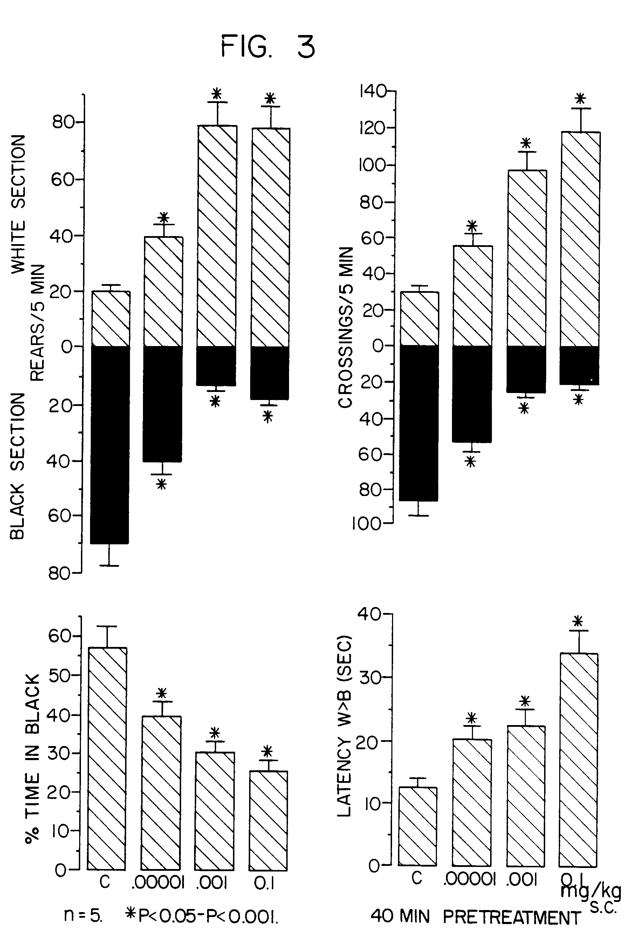

As shown in Figure 3, N-adamantyl-N'-o-tolyl-guanidine dose dependently increased the proportion of time the mice spent in the larger, lighted area of the test chamber. The numbers of line crossings and rears in the light compartment increased at the expense of those in the dark compartment. At the highest dose of N-adamantyl-N'-o-tolyl guanidine (0.1 mg/kg), the numbers of rears and line crossings did not decrease significantly showing that the drug was not sedative at this level. The latency to entering the dark compartment also increased in a dose dependant manner.

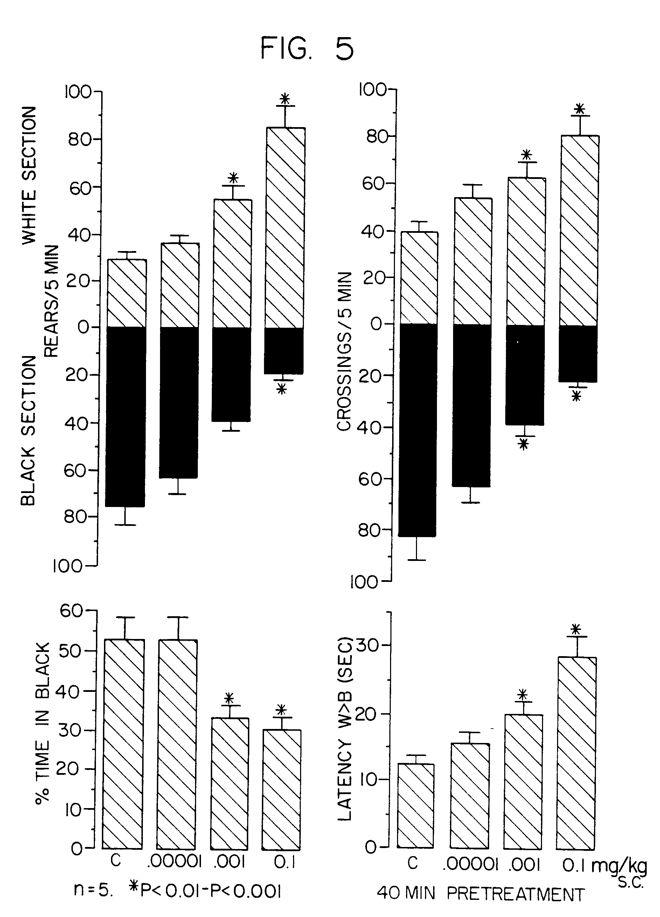

As shown in Figure 5, N-adamantyl-N'-o-iodophenyl-guanidine also increased, in a dose dependant manner, the proportion of time the mice spent in the larger, lighted area of the test chamber. The numbers of line crossings and rears in the light compartment increased at the expense of those in the dark compartment. At the highest dose of N-adamantyl-N'-o-iodophenyl guanidine (0.1 mg/kg), the numbers of rears and line crossings did not decrease significantly showing that the drug was not sedative at this level. The latency to entering the dark compartment also increased in a dose dependant manner.

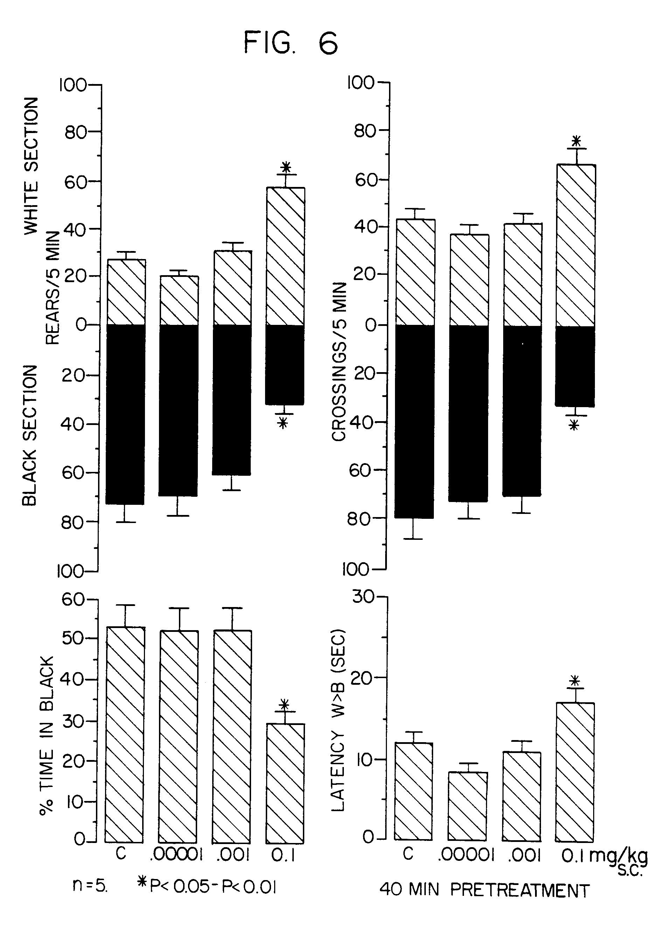

For comparison, a compound with a relatively low sigma receptor affinity was tested for anxiolytic activity. As shown in Figure 6, N-adamantyl-N'-{[(E)-2-phenylethenyl]phenyl}-guanidine (IC₅₀ = 1,000 nM) generally did not increase the proportion of time the mice spent in the larger, lighted area of the test chamber. The numbers of line crossings and rears in the light compartment tended to remain the same except at the highest dosage. At the highest dose of N-adamantyl-N'-{[(E)-2-phenylethenyl]phenyl} guanidine (0.1 mg/kg), the numbers of rears and line crossings did not decrease significantly showing that the drug was not sedative at this level.

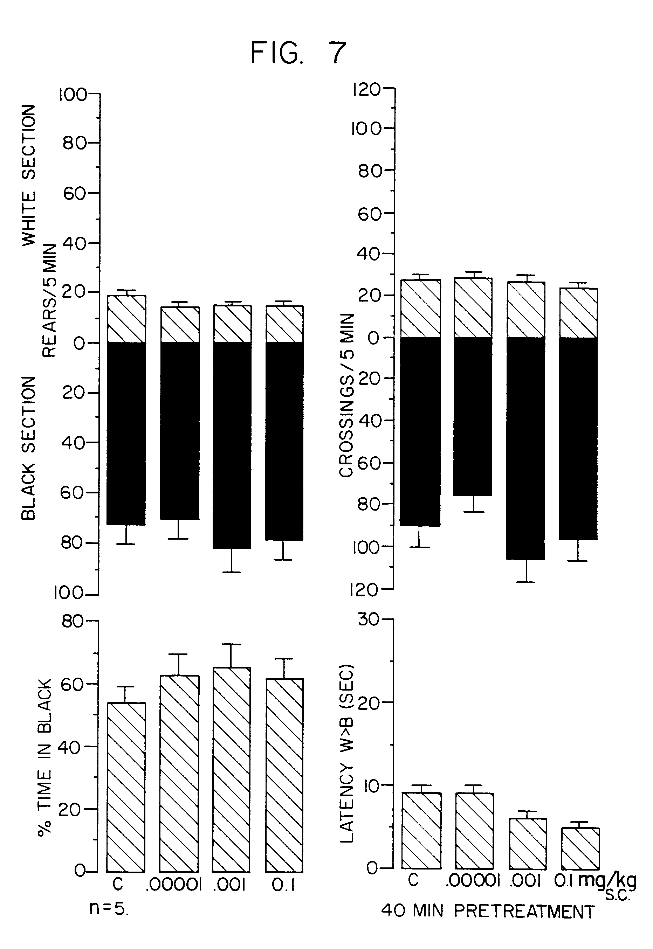

As shown in Figure 7, N,N'-di-o-tolylguanidine did not affect significantly the number of rears, crossings, or the % time in the black compartment at the concentrations tested. This result is surprising in light of the high sigma receptor binding of this compound (IC₅₀ = 28.0±1). Although the inventors do not wish to be bound by any particular theory, it would appear that DTG does not exhibit anxiolytic activity in the in vivo assay since it is quickly metabolized and thereby deactivated by the animal.

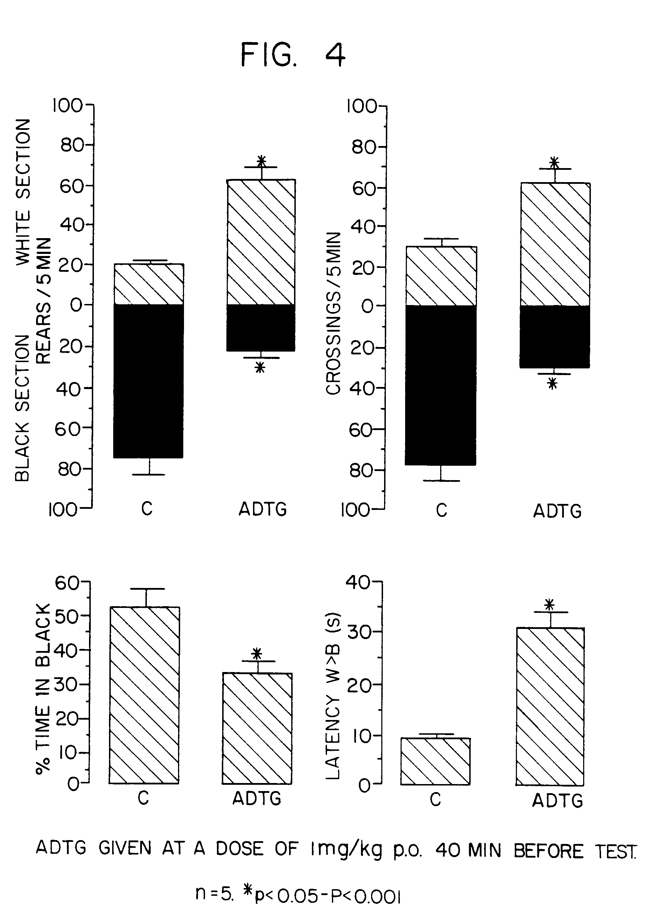

Next, N-adamantyl-N'-o-tolyl-guanidine, N,N-di-adamantylguanidine and N-adamantyl-N'-(o-iodophenyl)guanidine were orally administered to the mice at a dose of 1 mg/kg. As shown in Figure 4, orally administered N-adamantyl-N'-o-tolyl-guanidine increased significantly the proportion of time the mice spent in the larger, lighted area of the box. Moreover, the numbers of line crossings and rears in the light compartment increased at the expense of those in the dark compartment.

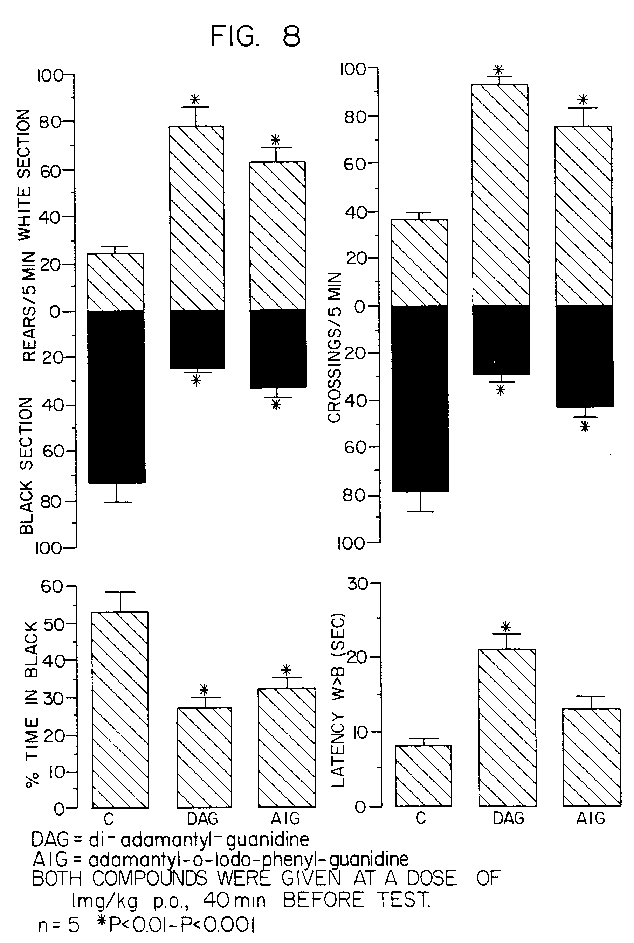

As shown in Figure 8, N,N-di-adamantylguanidine and N-adamantyl-N'-(o-iodophenyl)guanidine caused an increase in the numbers of line crossings and rears in the light compartment at the expense of those in the dark compartment. Compared with controls, the numbers of rears and line crossings did not decrease significantly showing that the two drugs were not sedative at the dosage level administered. The two drugs also caused an increase in the latency to entering the dark compartment in comparison to controls. These experiments confirm that N-adamantyl-N'-o-tolyl-guanidine, N,N-di-adamantylguanidine and N-adamantyl-N'-(o-iodophenyl)guanidine have anxiolytic activity when orally administered.

### Example 3 The Anxiolytic Potential of Diazepam and N-adamantyl-N'-o-tolylguanidine in a Rat Social Interaction Test

Male Hooded Lister rats (Glaxo bred, 200-250g), were housed 5 to a cage and were kept in the laboratory environment for at least a week before testing. Rats paired in the test were taken from separate cages.

The compounds were screened for anxiolytic activity by Brenda Costall according to the disclosure of Jones, B.J et al., Br. J. Pharmacol. 93:985-993 (1988) and File, S.E. et al., Br. J. Pharmacol. 62:19-24 (1978). The test arena consisted of an open-topped box, 62 x 62 x 33 cm with a 7 x 7 matrix of infra-red photocell beams in the walls, 2.5 cm from the floor. Diazepam and N-adamantyl-N'-o-tolyl guanidine were tested by treating both members of a pair of rats with the same treatment 40 min. before testing (Rats were placed singly in small cages immediately after dosing until they were tested).

Testing involved placing each member of a pair of rats in opposite corners of the arena and then leaving them undisturbed for 10 min. while recording their behavior remotely on videotape. The behavioral assessments were made subsequently from the recordings. The time spent in social interaction was measured and expressed as a cumulative total for the 10 min session. The behaviors that comprised social interaction were: following with contact, sniffing (but not sniffing of the hindquarters), crawling over and under, tumbling, boxing and grooming.

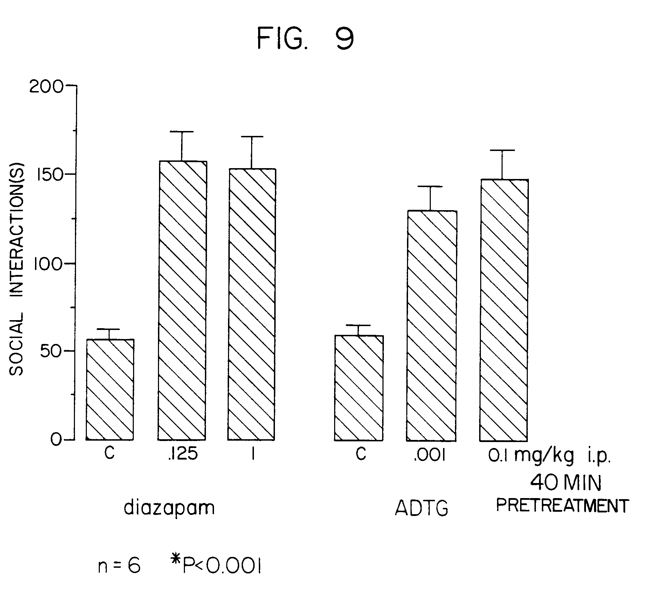

Intraperitoneally administered diazepam was tested over the dose range of 0.125-1 mg/kg. N-adamantyl-N'-o-tolylguanidine was tested over the range of 0.001-0.1 mg/kg. The results appear in Figure 9 (n = 6, P<0.001).

As shown in Figure 9, both diazepam and N-adamantyl-N'-o-tolylguanidine significantly increased social interactions. However, N-adamantyl-N'-o-tolylguanidine produced about the same result at one-tenth the dose of diazepam (1 mg/kg for diazepam and 0.1 mg/kg for N-adamantyl-N'-o-tolylguanidine). These results provide strong indication that N-adamantyl-N'-o-tolylguanidine will have anxiolytic activity in man, especially since the social interaction test in the rat is one of the most extensively validated tests (See File et al., supra).

## Claims

1. The use of an N,N'-disubstituted guanidine having the formula 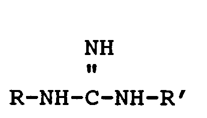 wherein R and R' are an alkyl group of at least 4 carbon atoms, a cycloalkyl group of at least 3 carbon atoms, a carbocyclic aryl group of at least 6 carbon atoms, alkaryl or aralkyl of at least 6 carbon atoms and containing 1-3 separate or fused rings, a heterocyclic ring, and wherein each of R and R' may be substituted in 1-3 positions, or wherein R and R' together with the guanidine group to which they are attached form a cyclic ring containing at least 2 carbon atoms exclusive of the guanidine carbon atom, and wherein said cyclic ring may be substituted with one or more alkyl groups of 1-6 carbon atoms, carbocyclic aryl groups of at least 6 carbon atoms, cycloalkyl groups of 3-12 carbon atoms, or 1-2 fused aromatic rings,
wherein said N,N'-disubstituted guanidine exhibits high affinity for the sigma receptor,
in the manufacture of an anxiolytic agent.

2. The use as claimed in claim 1, wherein said N,N'-disubstituted guanidine is selected from N-(2-iodophenyl)-N'-(adamant-1-yl)guanidine; N-(o-tolyl)-N'-(adamant-1-yl)guanidine; N,N'-di(adamant-1-yl)guanidine; N-cyclohexyl-N'-(2-methylphenyl)-guanidine; N-(adamant-1-yl)-N'-cyclohexylguanidine; N-(adamant-2-yl)-N'-(2-iodophenyl)guanidine; N-(adamant-2-yl)-N'-(2-methylphenyl)-guanidine; N-(exo-2-norbornyl)-N'-(2-methylphenyl)guanidine; N-(±)-endo-2-norbornyl)-N'-(2-methylphenyl)guanidine; N-(exo-2-norbornyl)-N'-(2-iodophenyl)guanidine; and N-((±)-endo-2-norbornyl)-N'-(2-iodophenyl)guanidine.

3. The use as claimed in claim 1, wherein said N,N'-disubstituted guanidine is N,N'-di-(adamant-1-yl)guanidine.

4. The use as claimed in claim 1, wherein said N,N'-disubstituted guanidine is N-(adamant-1-yl)-N'-(o-tolyl)guanidine.

5. The use as claimed in claim 1, wherein said N,N'-disubstituted guanidine is N-(adamant-1-yl)-N'-(o-iodophenyl) guanidine.

## Patentansprüche

1. Verwendung eines N,N'-disubstituierten Guanidins mit der Formel 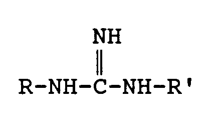 worin R und R' eine Alkylgruppe mit mindestens vier Kohlenstoffatomen, eine Cycloalkylgruppe mit mindestens drei Kohlenstoffatomen, eine carbocyclische Arylgruppe mit mindestens sechs Kohlenstoffatomen, eine Alkaryl- oder Aralkylgruppe mit mindestens sechs Kohlenstoffatomen und eins bis drei getrennten oder kondensierten Ringen oder einen heterocyclischen Ring bedeuten, wobei jeder der Reste R und R' in eins bis drei Positionen substitiuiert sein kann, oder worin R und R' zusammen mit der Guanidingruppe, mit der sie verknüpft sind, einen cyclischen Ring bilden, der mindestens zwei Kohlenstoffatome unter Ausschluß der Guanidin-Kohlenstoffatome aufweist, wobei der cyclische Ring mit einer oder mehreren Alkylgruppen mit eins bis sechs Kohlenstoffatomen, carbocyclischen Arylgruppen mit mindestens sechs Kohlenstoffatomen, Cycloakylgruppen mit drei bis zwölf Kohlenstoffatomen oder eins bis zwei kondensierten aromatischen Ringen substituiert sein kann,
wobei das N,N' disubstituierte Guanidin eine hohe Affinität für den Sigma-Rezeptor zeigt,
bei der Herstellung eines Anxiolytikums.

2. Verwendung nach Anspruch 1, wobei das N,N'-disubstituierte Guanidin unter N-(2-Iodphenyl)-N'-(adamant-1-yl)-guanidin; N-(o-Tolyl)-N'-(adamant-1-yl)-guanidin; N,N'-Di-(adamant-1-yl)-guanidin; N-Cyclohexyl-N'-(2-methylphenyl)-guanidin; N-(Adamant-1-yl)-N'-cyclohexylguanidin; N-(Adamant-2-yl)-N'-(2-iodphenyl)-guanidin; N-(Adamant-2-yl)-N'-(2-methylphenyl)-guanidin; N-(exo-2-Norbornyl)-N'-(2-methylphenyl)-guanidin; N-((±)-endo-2-Norbornyl)-N'-(2-methylphenyl)-guanidin; N-(exo-2-Norbornyl)-N'-(2-iodphenyl)-guanidin; und N-((±)-endo-2-Norbornyl)-N'-(2-iodphenyl)-guanidin ausgewählt ist.

3. Verwendung nach Anspruch 1, wobei es sich bei dem N,N'-disubstituierten Guanidin um N,N'-Di-(adamant-1-yl)-guanidin handelt.

4. Verwendung nach Anspruch 1, wobei es sich bei dem N,N'-disubstituierten Guanidin um N-(Adamant-1-yl)-N'-(o-tolyl)-guanidin handelt.

5. Verwendung nach Anspruch 1, wobei es sich bei dem N,N'-disubstituierten Guanidin um N-(Adamant-1-yl)-N'-(o-iodphenyl)-guanidin handelt.

## Revendications

1. Utilisation, pour la production d'un agent anxiolytique, d'une guanidine N,N'-disubstituée de formule:
R-NH-C(=NH)-NH-R' ,
dans laquelle R et R' représentent un groupe alkyle à au moins 4 atomes de carbone, un groupe cycloalkyle à au moins 3 atomes de carbone, un groupe aryle carbocyclique à au moins 6 atomes de carbone, un groupe alkaryle ou aralkyle à au moins 6 atomes de carbone et comportant 1 à 3 noyau(x), séparés ou condensés, ou un noyau hétérocyclique, et dans laquelle R et R' peuvent être chacun substitués sur 1 à 3 positions, ou bien dans laquelle R et R' forment, ensemble avec le groupe guanidine auquel ils sont liés, un noyau cyclique contenant au moins 2 atomes de carbone en plus de l'atome de carbone de la guanidine, et dans laquelle ledit noyau cyclique peut être substitué par un ou plusieurs groupe(s) alkyle en C₁₋₆, aryle carbocyclique à au moins 6 atomes de carbone ou cycloalkyle en C₃₋₁₂ ou par des noyaux aromatiques condensas en 1,2, dans laquelle ladite guanidine N,N'-disubstituée présente une force affinité pour le récepteur sigma.

2. Utilisation telle que revendiquée dans la revendication 1, dans laquelle ladite guanidine N,N'-disubstituée est choisie parmi les composés suivants: N-(2-iodophényl)-N'-(adamant-1-yl)guanidine, N-(o-tolyl)-N'-(adamant-1-yl)guanidine, N,N'-di(adamant-1-yl)guanidine, N-cyclohexyl-N'-(2-méthylphényl)-guanidine), N-(adamant-1-yl)-N'-cyclohexylguanidine, N-(adamant-2-yl)-N'-(2-iodophényl)guanidine, N-(adamant-2-yl)-N'-(2-méthylphényl)guanidine, N-(exo-2-norbornyl)-N'-(2-méthylphényl)guanidine, N-[(±)-endo-2-norbornyl]-N'-(2-méthylphényl)guanidine, N-(exo-2-norbornyl)-N'-(2-iodophényl)guanidine et N-[(±)-endo-2-norbornyl]-N'-(2-iodophényl)guanidine.

3. Utilisation telle que revendiquée dans la revendication 1, dans laquelle ladite guanidine N,N'-disubstituée est la N,N'-di(adamant-1-yl)guanidine.

4. Utilisation telle que revendiquée dans la revendication 1, dans laquelle ladite guanidine N,N'-disubstituée est la N-(o-tolyl)-N'-(adamant-1-yl)guanidine.

5. Utilisation telle que revendiquée dans la revendication 1, dans laquelle ladite guanidine N,N'-disubstituée est la N-(2-iodophényl)-N'-(adamant-1-yl)guanidine.
